# EUROPEAN PATENT APPLICATION

(11) **EP 3 499 228 A1**
(43) Date of publication of application: **19.06.2019**
(21) Application number: 17839611.5
(22) Date of filing: 14.08.2017
(51) Int. Cl.: G01N 33/48, C12Q 1/04, G01N 33/53, G01N 33/574

(54) **DETECTION METHOD OF CIRCULATING TUMOR CELLS AND PRETREATMENT METHOD FOR DETECTING CIRCULATING TUMOR CELLS**

(30) Priority: 12.08.2016 WO PCT/JP2016/073785; 09.09.2016 WO PCT/JP2016/076699; 09.09.2016 JP 2016177014
(71) Applicant: Hitachi Chemical Company, Ltd., Chiyoda-ku Tokyo 100-6606 (JP)
(72) Inventor: ENDOU Katsuya, Tokyo 100-6606 (JP); NAKAMURA Seita, Tokyo 100-6606 (JP); MATSUNAGA Tatsuya, Tokyo 100-6606 (JP); YAGI Satomi, Tokyo 100-6606 (JP); HIGUCHI Masayuki, Tokyo 100-6606 (JP); TSAI Anthony H., Irvine, CA 92617 (US)
(74) Representative: Hoffmann Eitle
(86) International application number: PCT/JP2017/029328
(87) International publication number: WO 2018/030547

(57) **Abstract**

The present invention provides a method for detecting circulating tumor cells, comprising steps of: capturing cells in a blood sample on a filter; contacting an antibody that is labeled with a first fluorescent dye and recognizes a leukocyte marker protein with the cell-captured filter; contacting an animal serum, a surfactant and an antibody that is labeled with a second fluorescent dye and recognizes an epithelial cell marker protein with the cell-captured filter; contacting a third fluorescent dye that stains nucleic acids with the cell-captured filter; and detecting fluorescences emitted from each cell captured on the filter.

## Description

### Technical Field

The present invention relates to a method for detecting circulating tumor cells and a pretreatment method for detecting circulating tumor cells.

### Background Art

As a method for predicting whether cancer has metastasized or not, there is a method which involves detecting circulating tumor cells (hereinafter also called "CTCs")that circulate in the body through the blood vessel and the lymphatic vessel. CTCs can be detected by first capturing CTCs on a filter (for example, Patent Literature 1) based on the difference in the size and the deformability of cells using the filter and then staining the captured CTCs.

### Citation List

### Patent Literature

Patent Literature 1: Japanese Unexamined Patent Publication No. 2013-42689

### Summary of Invention

### Technical Problem

Although the cells captured on the filter can be detected by subjecting the nuclei of the cells to fluorescent staining and detecting the fluorescence, leukocytes having a similar cell diameter as CTCs are also captured on the filter besides CTCs. Accordingly, whether the cells captured are leukocytes or CTCs can be determined by reacting fluorescence-labeled antibodies specific to leukocytes and CTCs, respectively, with the cells captured and detecting fluorescence that the cells emit. However, with conventional methods for detecting CTCs, there were cases where a fluorescence that indicates a nucleus of a cell and a fluorescence that indicates a CTC are observed in the captured leukocyte (false positive), or a fluorescence that indicates a nucleus of a cell, a fluorescence that indicates a leukocyte, and a fluorescence that indicates a CTC are observed in the captured cell (triple positive). As a result of examination by the present inventors, it has been found that when detecting CTCs in blood, the number of false positives and the number of triple positives vary depending on time after blood collection. When using fresh blood, more specifically, when using blood within 8 hours of blood collection, there were cases where triple positives occurred, but false positives did not occur or occurred only to the extent that caused no practical problems. Meanwhile, when using blood that were not fresh, more specifically, when using blood after 8 hours or more have passed from the blood collection, there were cases where both triple positives and false positives occurred to such an extent that they could cause practical problems. Although false positives can be suppressed by using fresh blood, in reality, fresh blood cannot be always provided. Therefore, the reduction of false positives and triple positives regardless of time after blood collection has been desired.

### Solution to Problem

The present inventors have earnestly examined in light of such circumstances and consequently found that treatment of cells captured on a filter with a surfactant and an animal serum is effective in reducing false positives and triple positives in the detection of CTCs, thereby completing the present invention.

The present invention provides a method for detecting circulating tumor cells in a blood sample, comprising steps of: (a) filtering a blood sample through a filter to capture cells on the filter; (b) contacting with the cell-captured filter, a primary antibody that recognizes a leukocyte marker protein, and subsequently a secondary antibody that recognizes the primary antibody and is labeled with a first fluorescent dye; (c) contacting (c1) an animal serum, (c2) a surfactant and (c3) an antibody that recognizes an epithelial cell marker protein and is labeled with a second fluorescent dye with the cell-captured filter simultaneously or in any order; (d) contacting a third fluorescent dye that stains nucleic acids with the cell-captured filter; and (e) irradiating the cell-captured filter with respective excitation lights of the first, second and third fluorescent dyes to detect respective fluorescences of the first, second and third fluorescent dyes emitted from each cell captured on the filter, wherein step (d) is performed at any stage between step (a) and step (e).

The present invention provides a method in which step (b) in the above-mentioned method is a step of contacting an antibody that recognizes a leukocyte marker protein and is labeled with a first fluorescent dye with the cell-captured filter.

Step (c) and step (d) may be performed simultaneously to contact (c1), (c2), (c3) and the third fluorescent dye that stains nucleic acids simultaneously with the cell-captured filter.

The surfactant may comprise a nonionic surfactant, or may comprise polyoxyethylene octyl phenyl ether. The concentration of the surfactant may be 0.05% by mass to 0.2% by mass.

An animal from which the animal serum derives, an animal from which the primary antibody or the antibody that recognizes a leukocyte marker protein derives, and an animal from which the antibody that recognizes an epithelial cell marker protein derives may be the same animal, and the animal may be a mouse. The concentration of the animal serum may be 2% by mass to 10% by mass.

The leukocyte marker protein may be CD45. The epithelial cell marker protein may be cytokeratin or a tumor marker.

The present invention provides a pretreatment method for detecting circulating tumor cells on a cell-captured filter, comprising a step of contacting an animal serum and a surfactant with the cell-captured filter simultaneously or in any order.

Prior to the step of contacting the animal serum and the surfactant, the cell-captured filter may be treated with a primary antibody that recognizes a leukocyte marker protein and a secondary antibody that recognizes the primary antibody and is labeled with a first fluorescent dye, or may be treated with an antibody that recognizes a leukocyte marker protein and is labeled with a first fluorescent dye.

The animal serum, the surfactant, an antibody that recognizes an epithelial cell marker protein and is labeled with a second fluorescent dye, and a third fluorescent dye that stains nucleic acids may be simultaneously contacted with the cell-captured filter.

The surfactant may comprise a nonionic surfactant, or may comprise polyoxyethylene octyl phenyl ether. The concentration of the surfactant may be 0.05% by mass to 0.2% by mass.

An animal from which the animal serum derives, an animal from which the primary antibody or the antibody that recognizes a leukocyte marker protein derives, and an animal from which the antibody that recognizes an epithelial cell marker protein derives may be the same animal, and the animal may be a mouse. The concentration of the animal serum may be 2% by mass to 10% by mass.

The leukocyte marker protein may be CD45. The epithelial cell marker protein may be cytokeratin or a tumor marker.

### Advantageous Effects of Invention

According to the present invention, false positives and triple positives in the detection of CTCs can be reduced.

### Brief Description of Drawings

Figure 1 is a perspective view showing one embodiment of a cell-capturing cartridge.
Figure 2 is a sectional view, taken from the II-II line in Figure 1.
Figure 3 is a graph showing the results of triple positives in Test Example 1.
Figure 4 is a graph showing the results of false positives in Test Example 1.
Figure 5 is a graph showing the results of background staining in Test Example 1.
Figure 6 is a graph showing the results of triple positives in Test Example 2.
Figure 7 is a graph showing the results of false positives in Test Example 2.
Figure 8 is a graph showing the results of background staining in Test Example 2.
Figure 9 is a graph showing the results of triple positives in Test Example 3.
Figure 10 is a graph showing the results of false positives in Test Example 3.
Figure 11 is a graph showing the results of background staining in Test Example 3.
Figure 12 is a graph showing a temporal change in the number of false positives when healthy human blood was used.
Figure 13 is a graph showing a temporal change in the number of triple positives when healthy human blood was used.

### Description of Embodiments

A method for detecting circulating tumor cells in a blood sample according to one embodiment of the present invention comprises steps of: (a) filtering a blood sample through a filter to capture cells on the filter; (b) contacting with the filter having the cells captured thereon, a primary antibody that recognizes a leukocyte marker protein, and subsequently a secondary antibody that recognizes the primary antibody and is labeled with a first fluorescent dye; (c) contacting (c1) an animal serum, (c2) a surfactant and (c3) an antibody that recognizes an epithelial cell marker protein and is labeled with a second fluorescent dye with the cell-captured filter simultaneously or in any order; (d) contacting a third fluorescent dye that stains nucleic acids with the cell-captured filter; and (e) irradiating the cell-captured filter with respective excitation lights of the first, second and third fluorescent dyes to detect respective fluorescences of the first, second and third fluorescent dyes emitted from each cell captured on the filter. These steps will be described in detail hereinafter.

First, in step (a), a blood sample is filtered through a filter to capture cells in the blood sample on the filter. A "cell" used herein means a leukocyte or a circulating tumor cell (CTC) unless otherwise specified. Although CTCs are not contained in the blood of a healthy person, CTCs are contained in the blood of a subject whose cancer has metastasized, and therefore when the blood of a subject whose cancer has metastasized is filtered through a filter, CTCs are captured on the filter. Since the diameter of leukocytes is similar to the diameter of CTCs, leukocytes are captured together with CTCs on a filter.

As the blood sample, blood collected from a subject may be used as it is, and blood diluted with a buffer solution such as phosphate buffered saline (PBS) or other suitable media may also be used. Additives such as an anticoagulant and a fixing agent usually added to blood samples may be added to the blood sample.

The filter is not particularly limited as long as it is a filter which can selectively capture leukocytes and CTCs existing in a blood sample, and conventionally known filters may be used. The filter may be a metal filter, and has through pores having a pore size of preferably 5 µm to 15 µm, more preferably 6 µm to 12 µm, and further preferably 7 µm to 10 µm. The pore size of the through pores means a maximum diameter of a sphere that can pass through the through pores.

The cell-captured filter may be washed after step (a) (step (x)). Step (x) is performed, for example, by contacting a cleaning liquid comprising a known buffer solution such as PBS with the filter. An additive such as bovine serum albumin (BSA) or ethylenediaminetetraacetic acid (EDTA) may be contained in the cleaning liquid. Step (x) may be performed not only after step (a), but also after each step as appropriate.

"Contacting" of a substance with the cell-captured filter herein may be performed by passing the substance or a solution of the substance through the cell-captured filter or immersing the cell-captured filter in the substance or a solution of the substance, but is not limited to these methods.

Next, in step (b), a primary antibody that recognizes a leukocyte marker protein may be contacted with the cell-captured filter, and a secondary antibody that recognizes the primary antibody and is labeled with a first fluorescent dye may be subsequently contacted. The leukocytes captured on the filter are fluorescence-labeled by this step. After contacting the primary antibody with the cell-captured filter, the cell-captured filter may be washed with a cleaning liquid (step (x)) prior to contacting secondary antibody.

Although leukocytes captured on the filter may be fluorescence-labeled in two steps as mentioned above, they may also be fluorescence-labeled in one step. That is, in step (b), the leukocytes captured on the filter may be fluorescence-labeled in one step by contacting an antibody that recognizes a leukocyte marker protein and is labeled with a first fluorescent dye with the cell-captured filter.

Examples of the leukocyte marker protein include CD45, which is expressed on all hematopoietic stem cells.

The primary antibody that recognizes a leukocyte marker protein, the secondary antibody labeled with a first fluorescent dye, and the antibody that recognizes a leukocyte marker protein and is labeled with a first fluorescent dye are not limited, and may each be a polyclonal antibody or a monoclonal antibody. Animals from which the antibodies derive are not limited as long as an animal from which the primary antibody derives and an animal from which the secondary antibody derives are different.

The first fluorescent dye is not particularly limited as long as it is a fluorescent dye usually used for the fluorescence labeling of antibodies. The first fluorescent dye is different from the second and third fluorescent dyes. Since the fluorescent dyes have different fluorescence wavelengths, they can be distinguished. As the first fluorescent dye, for example, Alexa Fluor (registered trademark) may be used.

The cells captured on the filter may be fixed after step (b) (step (y1)). The cells may be fixed by contacting a known fixing agent such as formaldehyde with the cell-captured filter. By fixing the cells, the decomposition or the aggregation of the cells can be reduced further.

Further, the cells captured on the filter may be permeabilized after step (y1) (step (y2)). The cells may be permeabilized by contacting a known permeabilization agent with the cell-captured filter. As the permeabilization agent, for example, polyoxyethylene octyl phenyl ether may be used.

It is preferable that steps (y1) and (y2) be performed between step (b) and step (c).

Next, in step (c), (c1) an animal serum, (c2) a surfactant and (c3) an antibody that recognizes an epithelial cell marker protein and is labeled with a second fluorescent dye may be contacted with the cell-captured filter.

Contacting the animal serum (c1) with the cell-captured filter acts to reduce false positives and triple positives in the detection of CTCs. Although the mechanism in which false positives and triple positives are reduced is not certain, it is speculated that it is because the unspecific bindings of the antibodies can be reduced by contacting the animal serum with the cells.

Although the animal serum (c1) is not particularly limited as long as it is a commonly used animal serum, it may be a serum derived from the same animal as an animal from which the antibody that recognizes an epithelial cell marker protein derives, and may be a serum derived from the same animal as an animal from which the antibody that recognizes an epithelial cell marker protein derives, and an animal from which the primary antibody or the antibody that recognizes a leukocyte marker protein derives. In the latter case, the method of the present invention is particularly effective in a reduction of false positives and triple positives. For example, when the primary antibody or the antibody that recognizes a leukocyte marker protein and the antibody that recognizes an epithelial cell marker protein are antibodies derived from a mouse, the animal serum is preferably a serum derived from a mouse.

The animal serum (c1) may be diluted with a buffer solution such as PBS or other suitable media. The concentration of the animal serum (c1) is preferably 2% by mass to 10% by mass, more preferably 4% by mass to 6% by mass, and further preferably 5% by mass. When the concentration of the animal serum is 2% by mass or more, false positives and triple positives are reduced further in the case where it is used together with the below-mentioned surfactant. When the concentration of the animal serum is 10% by mass or less, the contamination of the filter by the animal serum is reduced in the case where it is used together with the below-mentioned surfactant.

Contacting the surfactant (c2) with the cell-captured filter acts to reduce false positives and triple positives in the detection of CTCs. Although the mechanism in which false positives and triple positives are reduced is not certain, it is speculated that it is because nonspecific bindings of the antibodies can be reduced by contacting the surfactant with the cells.

Moreover, even if the filter is contaminated by the adhesion of the animal serum (c1) to the filter, the animal serum adhered to the filter is removed with the surfactant by contacting the surfactant (c2) with the filter.

It is preferable that the surfactant comprises a nonionic surfactant. Examples of the nonionic surfactant include polyoxyethylene octyl phenyl ether, polyethylene glycol sorbitan monolaurate (also called polysorbate 20) and n-octyl-β-D-glucopyranoside (also called octyl glucoside), and polyoxyethylene octyl phenyl ether is preferable.

The surfactant (c2) may be diluted with a buffer solution such as PBS or other suitable media. The concentration of the surfactant is preferably 0.05% by mass to 0.2% by mass, more preferably 0.05% by mass to 0.1% by mass, and further preferably 0.05% by mass. When the concentration of the surfactant is 0.05% by mass or more, false positives and triple positives are reduced further in the case where it is used together with the animal serum. When the concentration of the surfactant is 0.2% by mass or less, the physical shapes of the cells are well maintained.

Examples of the epithelial cell marker protein which is the antigen of the antibody (c3) that recognizes an epithelial cell marker protein and is labeled with a second fluorescent dye include cytokeratin, epithelial cell adhesion molecules (EpCAMs), CD146, CD176 and tumor markers such as EGFR and HER2, and cytokeratin or the tumor markers are preferable. Since CTCs are derived from epithelial cells, they comprise these epithelial cell marker proteins. The second fluorescent dye is not particularly limited as long as it is a fluorescent dye usually used for the fluorescence labeling of antibodies. As the second fluorescent dye, for example, fluorescein such as fluorescein isothiocyanate (FITC) may be used.

The antibody (c3) is not particularly limited, and may be a polyclonal antibody or a monoclonal antibody. The animal from which the antibody (c3) derives is not limited.

In step (c), (c1), (c2) and (c3) may be contacted with the cell-captured filter simultaneously or in any order. From the viewpoint of producing the effect of the present invention more remarkably, it is preferable to contact (c1) and (c2) simultaneously and subsequently contact (c3), and it is more preferable to contact (c1), (c2) and (c3) simultaneously. In step (c), washing step (x) may be performed as appropriate.

Next, in step (d), a third fluorescent dye that stains nucleic acids may be contacted with the cell-captured filter.

The third fluorescent dye that stains nucleic acids is not particularly limited as long as it is a fluorescent dye which can bind to nucleic acids, and a fluorescent dye usually used for the fluorescence staining of nucleic acids may be used. Examples of the third fluorescent dye include 4',6-diamidino-2-phenylindole (DAPI) and 2'-(4-ethoxy phenyl)-5-(4-methyl-1-piperazinyl)-2,5'-bi-1H-benzimidazole trihydrochloride (Hoechst 33342).

Although step (d) may be performed right after step (c), it is not essential to perform it right after step (c), and it may be performed at any stage between step (a) and step (e). It is preferable to perform step (c) and step (d) simultaneously from the viewpoint of producing an effect of the present invention more remarkably. More specifically, it is preferable to contact (c1) and (c2) with the cell-captured filter simultaneously, and subsequently contact (c3) and the third fluorescent dye that stains nucleic acids simultaneously, and it is more preferable to contact (c1), (c2), (c3) and the third fluorescent dye that stains nucleic acids simultaneously.

When the animal serum (c1) and the surfactant (c2) is contacted with the cell-captured filter simultaneously, a mixed solution obtained by mixing the animal serum and the surfactant beforehand may be used. For the combination of the concentrations of the animal serum and the surfactant in the mixed solution, 2% by mass to 10% by mass of the animal serum and 0.05% by mass to 0.2% by mass of the surfactant is preferable, 4% by mass to 6% by mass of the animal serum and 0.05% by mass to 0.1% by mass of the surfactant is more preferable, 5% by mass of the animal serum and 0.05% by mass to 0.1% by mass of the surfactant is further preferable, and 5% by mass of the animal serum and 0.05% by mass of the surfactant is particularly preferable. When the combination of the concentrations of the animal serum and the surfactant is in the above-mentioned range, an effect of the present invention can be produced more remarkably. Moreover, background staining due to the contamination of the filter can be reduced, and the physical shapes of the cells can be maintained.

When only the animal serum among the animal serum (c1) and the surfactant (c2) is contacted with the cell-captured filter, false positives and triple positives cannot be sufficiently reduced, or the animal serum adheres to the filter (the filter is contaminated). When the animal serum adheres to the filter, the secondary antibody or the antibody that is present on the filter and is labeled with a first fluorescent dye binds to the adhered animal serum (background staining), and fluorescence of the first fluorescent dye is detected in the wide area of the filter thereby in step (e), which makes the detection of CTCs difficult. When only the surfactant among the animal serum (c1) and the surfactant (c2) is contacted with the cells, false positives and triple positives cannot be reduced sufficiently or the physical shapes of the cells captured are destroyed, which makes the detection of CTCs difficult. According to the invention of the present embodiment, false positives and triple positives are reduced sufficiently, background staining due to the contamination of the filter is reduced, and the physical shapes of the cells are maintained, by contacting the combination of the animal serum and the surfactant with the cell-captured filter.

Finally, in step (e), the cell-captured filter is irradiated with respective excitation lights of the first, second and third fluorescent dyes to detect respective fluorescences of the first, second and third fluorescent dyes emitted from each cell captured on the filter.

Leukocytes are labeled with the first and third fluorescent dyes. Therefore, upon detecting the fluorescences of the first, second and third fluorescent dyes, cells in which the fluorescence of the second fluorescent dye is not detected (negative), but the fluorescences of the first and third fluorescent dyes are detected (positive) are identified as leukocytes. Meanwhile, CTCs are labeled with the second and third fluorescent dyes. Therefore, cells in which fluorescence of the first fluorescent dye is not detected (negative), but the fluorescences of the second and third fluorescent dyes are detected (positive) are identified as CTCs. Here, when the first, second and third fluorescent dyes are detected (positive), namely in the case of a triple positive, the cell cannot be identified as which of a leukocyte and a CTC. False positive is a case where, the fluorescence of the first fluorescent dye is not detected (negative), but the fluorescences of the second and third fluorescent dyes are detected (positive), even though the cell is a leukocyte.

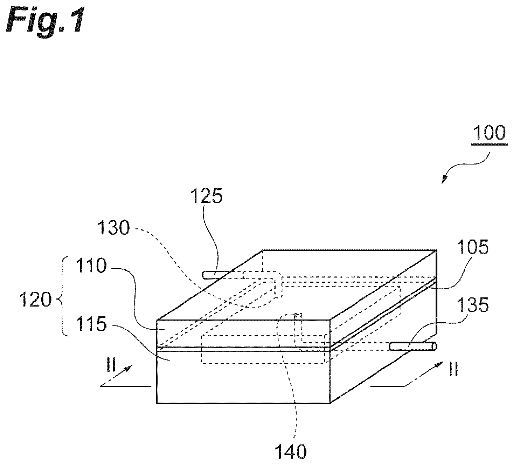

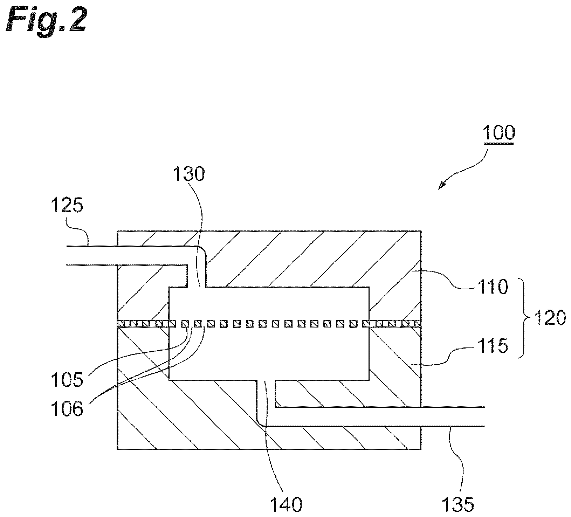

Next, a method for detecting CTCs in a blood sample according to another embodiment of the present invention will be described with reference to Figure 1 and Figure 2. Details of the filter, the primary antibody that recognizes a leukocyte marker protein, the secondary antibody labeled with a first fluorescent dye, the antibody that recognizes a leukocyte marker protein and is labeled with a first fluorescent dye, (c1) the animal serum, (c2) the surfactant, (c3) the antibody labeled with a second fluorescent dye, the third fluorescent dye that stains nucleic acids, and other reaction solutions in the method according to the present embodiment are as described in the above-mentioned embodiment.

A CTC-capturing cartridge (cartridge) 100 shown in Figure 1 and Figure 2 comprises: a case 120 comprising an inflow port 130 to which an inflow pipe 125 into which liquid flows is connected, and an outflow port 140 to which an outflow pipe 135 out of which liquid flows is connected; and a filter 105. The filter 105 is fixed with the case 120 comprising an upper member 110 and a lower member 115. A blood sample, a cleaning liquid and other reaction solutions are introduced through the inflow pipe 125 into the inside of the case 120, pass through the filter 105, and are discharged from the outflow pipe 135 outside. Such a liquid flow may be generated, for example, by connecting a pump to the upstream of the inflow pipe 125 or to the downstream of the outflow pipe 135. A cock may be provided at the upstream of the inflow pipe 125 and/or the downstream of outflow pipe 135 to regulate the liquid flow.

First, a blood sample is introduced from the inflow pipe 125 into the cartridge 100 and filtered through the filter 105 (step (a)). Leukocytes and CTCs in the blood sample cannot pass through pores 106 of the filter 105, and remain on the surface of the filter 105. The other components in the blood sample pass through the pores 106, and are discharged out of the cartridge 100. Subsequently, the filter 105 may be washed by passing a cleaning liquid through the filter 105 (step (x)). Washing step (x) may be performed after the following steps as appropriate.

Next, the cells captured on the filter 105 may be reacted with a primary antibody that recognizes a leukocyte marker protein by introducing a solution comprising the primary antibody into the cartridge 100 and retaining it in the cartridge 100 for a predetermined period of time. Subsequently, the primary antibody may be reacted with a secondary antibody labeled with a first fluorescent dye by introducing a solution comprising the secondary antibody into the cartridge 100 and retaining it in the cartridge 100 for a predetermined period of time (step (b)). After the cells and the primary antibody are reacted and before the primary antibody and the secondary antibody are reacted, the filter 105 may be washed by passing a cleaning liquid through the filter 105 (step (x)).

Although step (b) may be performed in two steps using the primary antibody that recognizes a leukocyte marker protein and the secondary antibody labeled with a first fluorescent dye as mentioned above, it may also be performed in one step using an antibody that recognizes a leukocyte marker protein and is labeled with a first fluorescent dye. When performing step (b) in one step, the cells captured on the filter 105 and the antibody that recognizes a leukocyte marker protein and is labeled with a first fluorescent dye are reacted by introducing a solution comprising the antibody into the cartridge 100 and retaining it in the cartridge 100 for a predetermined period of time.

Here, the cells captured on the filter 105 may be fixed by introducing a solution comprising a fixing agent into the cartridge 100 and retaining it in the cartridge 100 for a predetermined period of time (step (y1)). Subsequently, the cells captured on the filter 105 may be permeabilized by introducing a solution comprising a permeabilization agent into the cartridge 100 and retaining it in the cartridge 100 for a predetermined period of time (step (y2)).

Next, the cells captured on filter 105 may be reacted with an animal serum (c1), a surfactant (c2) and an antibody (c3) labeled with a second fluorescent dye by introducing a solution comprising (c1), a solution comprising (c2) and a solution comprising (c3) into the cartridge 100 and retaining them in the cartridge 100 for a predetermined period of time (step (c)). The solutions may be introduced into the cartridge 100 separately, or mixed solutions obtained by mixing solutions in any combination may be introduced into the cartridge 100 in any order.

Next, the cells captured on the filter 105 may be reacted with a third fluorescent dye that stains nucleic acids by introducing a solution comprising the third fluorescent dye that stains nucleic acids into the cartridge 100 and retaining it in the cartridge 100 for a predetermined period of time (step (d)). Although step (d) may be performed right after step (c), it is not essential to perform it right after step (c), and it may be performed at any stage between step (a) and step (e). The solution comprising the third fluorescent dye that stains nucleic acids may be mixed with solutions of step (c) in any combination, and may be introduced into the cartridge 100 as a mixed solution.

Finally, by using a fluorescence microscope, the cartridge 100 is irradiated with the respective excitation lights of the fluorescent dyes and fluorescences emitted from each cell captured on the filter 105 (step (e)) are detected. The fluorescences are detected, for example, by observing the cartridge 100 from the top surface in the perpendicular direction of the cartridge 100 and processing fluorescence observation images. A cell is identified as which of a leukocyte and a CTC depending on the combination of the detected fluorescences.

A pretreatment method for detecting circulating tumor cells on a cell-captured filter according to one embodiment of the present invention comprises a step of contacting an animal serum and a surfactant with the cell-captured filter simultaneously or in any order. The details of the filter, the animal serum, the surfactant and the other reaction solutions in the method according to the present embodiment are as described in the embodiments of the method for detecting circulating tumor cells in a blood sample.

The cell-captured filter may be obtained, for example, by filtering a blood sample through a filter so as to capture cells on a filter. The cell-captured filter may, in advance, be contacted with a primary antibody that recognizes a leukocyte marker protein first and subsequently with a secondary antibody that recognizes the primary antibody and is labeled with a first fluorescent dye, or may, in advance, be contacted with an antibody that recognizes a leukocyte marker protein and is labeled with a first fluorescent dye. The cell-captured filter may further be contacted with a third fluorescent dye that stains nucleic acids.

The obtained cell-captured filter may be pretreated by contacting the animal serum and the surfactant with the cell-captured filter. Although the animal serum and the surfactant may be contacted with the cell-captured filter simultaneously or in any order, it is preferable to contact them simultaneously from the viewpoint of producing an effect of the present invention more remarkably.

After the pretreatment method according to the present embodiment is performed on the cell-captured filter, circulating tumor cells are detected. Circulating tumor cells may be detected, for example, as follows. First, an antibody that recognizes an epithelial cell marker protein and is labeled with a second fluorescent dye, and a third fluorescent dye that stains nucleic acids are contacted with the pretreated cell-captured filter simultaneously or in any order. However, if the cell-captured filter has, in advance, been contacted with the third fluorescent dye that stains nucleic acids prior to the pretreatment, the third fluorescent dye that stains nucleic acids does not need to be contacted with the filter again at this stage. Subsequently, the excitation lights of the first, second and third fluorescent dyes are each irradiated, and the fluorescences of the first, second and third fluorescent dyes emitted from each cell captured on the filter are detected. Cells which are negative for the first fluorescent dye and positive for the second and third fluorescent dyes are identified as circulating tumor cells.

Here, the antibody labeled with a second fluorescent dye and the third fluorescent dye that stains nucleic acids, both of which are contacted with the cell-captured filter after the pretreatment, may alternatively be contacted with the cell-captured filter beforehand in the stage of the pretreatment. That is, in the pretreatment method according to the present embodiment, the antibody labeled with a second fluorescent dye, and the third fluorescent dye that stains nucleic acids may be simultaneously contacted with the cell-captured filter together with the animal serum and the surfactant.

According to the pretreatment method according to the present embodiment, triple positives and false positives can be reduced in the detection of CTCs.

### Examples

### <Test Example 1>

### (Example 1)

CTCs in a blood sample were detected as follows using a CTC-capturing cartridge (cartridge) into which a metallic filter, which was a thin film comprising many through pores having a major axis of 100 µm and a minor axis of 8 µm (6 mm × 6 mm in film area, and 18 µm in film thickness), was incorporated. The CTC-capturing cartridge corresponds to the cartridge 100 described in the above-mentioned embodiment. The steps from step (a) to step (c) were performed using the CTC-capturing device. The CTC-capturing device comprises a reservoir into which a blood sample and other reaction solutions are introduced.

First, the cartridge was filled with a PBS solution containing 0.5% of BSA and 2 mM of EDTA (hereinafter also called "cleaning liquid"). The reservoir was charged with 7 mL of a cleaning liquid, and 3 mL of blood from a healthy human collected in the Cell Free DNA Blood Collection Tube of Streck, Inc. was added under the cleaning liquid so that the blood and the cleaning liquid formed layers. The CTC-capturing device was operated, the blood and the cleaning liquid in the reservoir were introduced into the cartridge at a flow rate of 200 µL/minute to capture leukocytes in blood on the filter. The cleaning liquid was introduced into the cartridge to wash off blood components remaining on the filter.

Into the cartridge, 1.25 mL of an anti-human CD45 mouse monoclonal antibody clone: 2D1 was introduced at a flow rate of 200 µL/minute and reacted at room temperature for 30 minutes. Into the cartridge, 1.40 mL of the cleaning liquid was introduced at a flow rate of 400 µL/minute to discharge the reaction solution in the cartridge. Into the cartridge, 1.25 mL of an Alexa Fluor (registered trademark) 594-labeled anti-mouse IgG goat polyclonal antibody was introduced at a flow rate of 400 µL/minutes and reacted at room temperature for 30 minutes. Into the cartridge, 1.40 mL of the cleaning liquid was introduced at a flow rate of 400 µL/mimite to discharge the reaction solution in the cartridge.

Into the cartridge, 1.25mL of a PBS solution containing 0.5% by mass to 4% by mass of formaldehyde was introduced at a flow rate of 400 µL/minute and reacted at room temperature for 10 minutes to fix the cells. Into the cartridge, 1.40 mL of the cleaning liquid was introduced at a flow rate of 400 µL/minute to discharge the reaction solution in the cartridge.

Into the cartridge, 1.25 mL of a PBS solution containing 0.05% by mass to 0.1% by mass of Triton X-100 (manufactured by Sigma-Aldrich Inc.) was introduced at a flow rate of 400 µL/minute and reacted at room temperature for 10 minutes to permeabilize the cells. Into the cartridge, 1.40 mL of the cleaning liquid was introduced at a flow rate of 400 µL/minute to discharge the reaction solution in the cartridge.

Into the cartridge, 1.25 mL of a solution (hereinafter also called "solution C") containing a mixture of FITC-labeled anti-human cytokeratin mouse monoclonal antibody clones: CK3/6H5/AE1/AE3, DAPI, 5% by mass of a mouse serum, 0.05% by mass of Triton X-100 and the cleaning liquid was introduced at a flow rate of 400 µL/minute and reacted at room temperature for 30 minutes. Into the cartridge, 3.00 mL of the cleaning liquid was introduced at a flow rate of 400 µL/minute to discharge the reaction solution in a cartridge. Subsequently, the cartridge was removed from the CTC-capturing device.

The cartridge was placed under a fluorescence microscope. The fluorescent dyes (FITC, Alexa Fluor 594 and DAPI) on the cells were excited using a fluorescence mirror unit. Photographs of fluorescences emitted from each fluorescent dye were taken, and the obtained images were merged. Cells exhibiting triple positive and cells exhibiting false positive were extracted from the merged image visually or using image-analyzing software, and the respective numbers of the cells were determined. The results are shown in Table 1. Here, cells exhibiting triple positive are cells which are positive for FITC, positive for Alexa Fluor 594 and positive for DAPI. Cells exhibiting false positive are cells which are positive for FITC, negative for Alexa Fluor 594 and positive for DAPI.

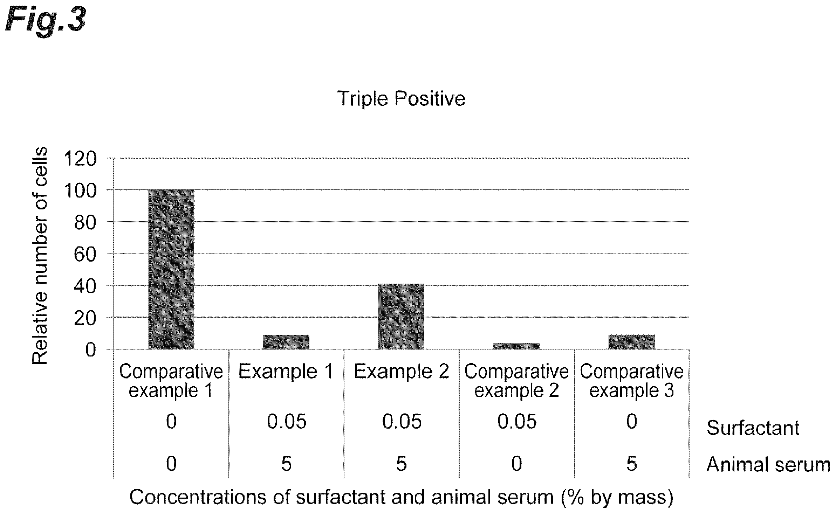

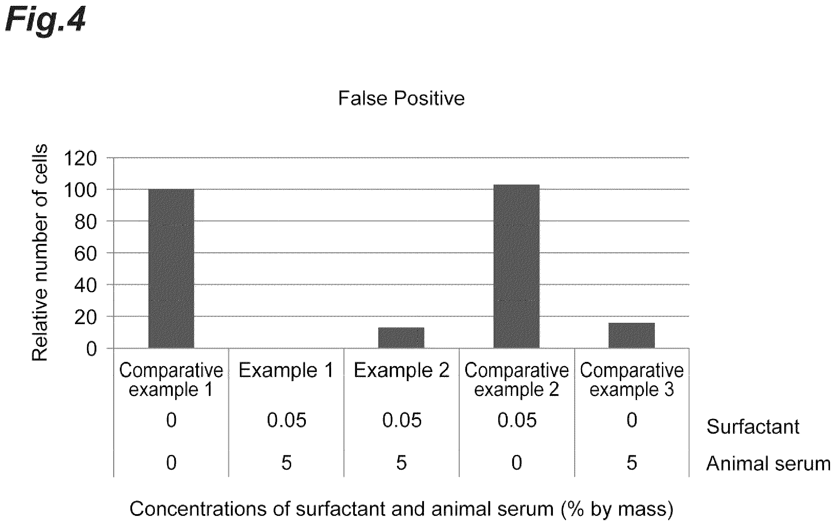

The number of cells exhibiting triple positive is expressed as a value relative to the number of cells exhibiting triple positive in Comparative Example 1, taken as 100. The number of cells exhibiting false positive is similarly expressed as a value relative to the number of cells exhibiting false positive in Comparative Example 1, taken as 100. The results are shown in Table 1, Figure 3 and Figure 4.

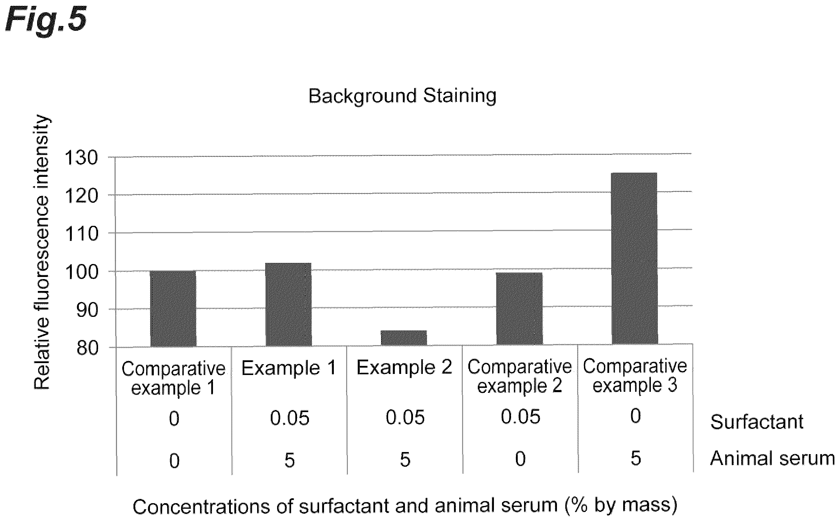

The staining of the filter (background staining) was evaluated as follows. Fluorescence intensity was measured as spots at regions in the upper right, the lower right, the upper left, the lower left and the center of the filter where cells do not exist. The obtained fluorescence intensity is expressed as a value relative to the fluorescence intensity of background staining in Comparative Example 1, taken as 100. The results are shown in Table 1 and Figure 5.

The physical shape of the cells were evaluated as follows. A plurality of cells captured on the filter were sampled at random, and the shape of the cells were observed visually. The results are shown in Table 1. In Table 1, A indicates a case where the deformation of the cells was not observed, and B indicates a case where a slight deformation of the cells was observed, but only to the extent not problematic for the observation of the cells.

### (Comparative Example 1)

The number of cells exhibiting triple positive, the number of cells exhibiting false positive and the fluorescence intensity of background staining were determined, and the physical shape of the cells were evaluated in the same way as in Example 1 except that a solution C not containing a mouse serum or Triton X-100 was used. The results are shown in Table 1.

### (Example 2)

Steps up to the permeabilization of cells were performed in the same way as in Example 1. Then, 1.25 mL of a solution containing 5% by mass of the mouse serum, 0.05% by mass of Triton X-100 and the cleaning liquid was introduced into the cartridge at a flow rate of 400 µL/minute and reacted at room temperature for 30 minutes. Into the cartridge, 1.50 mL of the cleaning liquid was introduced at a flow rate of 400 µL/minute to discharge the reaction solution in the cartridge. Into the cartridge, 1.25 mL of a PBS solution containing Anti-Cytokeratin-FITC and DAPI was introduced at a flow rate of 400 µL/minute and reacted at room temperature for 30 minutes. Into the cartridge, 3.00 mL of the cleaning liquid was introduced at a flow rate of 400 µL/minute to discharge the reaction solution in the cartridge. Subsequently, the cartridge was removed from the CTC-capturing device. Then, the relative value of the number of cells exhibiting triple positive, the relative value of the number of cells exhibiting false positive and the relative value of the fluorescence intensity of background staining were determined, and the physical shape of the cells were evaluated in the same way as in Example 1. The results are shown in Table 1 and Figure 3 to Figure 5.

### (Comparative Example 2)

The relative value of the number of cells exhibiting triple positive, the relative value of the number of cells exhibiting false positive and the relative value of the fluorescence intensity of background staining were determined, and the physical shape of the cells were evaluated in the same way as in Example 1 except that a solution C not containing a mouse serum was used. The results are shown in Table 1 and Figure 3 to Figure 5.

### (Comparative Example 3)

The relative value of the number of cells exhibiting triple positive, the relative value of the number of cells exhibiting false positive and the relative value of the fluorescence intensity of background staining were determined, and the physical shape of the cells were evaluated in the same way as in Example 1 except that a solution C not containing Triton X-100 was used. The results are shown in Table 1 and Figure 3 to Figure 5.

**[Table 1]**

| | Concentration (% by mass) | | Result | | | | | | |
|---|---|---|---|---|---|---|---|---|---|
| | Surfactant | Animal serum | Triple positive | | False positive | | Background staining | | Cell deformation |
| | | | Number of cells | Relative value | Number of cells | Relative value | Fluorescence intensity | Relative value | |
| Comparative Example 1 | 0 | 0 | 210 | - | 32 | - | 24.54 | - | A |
| Example 1 | 0.05 | 5 | 19 | 9 | 0 | 0 | 25.15 | 102 | A |
| Example 2 | 0.05 | 5 | 87 | 41 | 4 | 13 | 20.71 | 84 | A |
| Comparative Example 2 | 0.05 | 0 | 8 | 4 | 33 | 103 | 24.32 | 99 | A |
| Comparative Example 3 | 0 | 5 | 18 | 9 | 5 | 16 | 30.72 | 125 | A |

When the animal serum and the surfactant were reacted with the cells (Examples 1 and 2), triple positives and false positives were reduced compared to Comparative Example 1. In addition, background staining was reduced, and the deformation of cells was not observed either. Meanwhile, when only either one of the animal serum and the surfactant was reacted with cells (Comparative Examples 2 and 3), false positives were not reduced sufficiently (Comparative Example 2), or the staining intensity of background staining increased greatly (Comparative Example 3). When the animal serum and the surfactant were reacted with the cells together with the anti-cytokeratin antibody and the nucleic acid-staining agent (Example 1), triple positives and false positives were reduced further than those when the animal serum and the surfactant were reacted with the cells before the anti-cytokeratin antibody and the nucleic acid-staining agent were reacted (Example 2).

### <Test Example 2>

### (Example 3 and Comparative Examples 4 to 6)

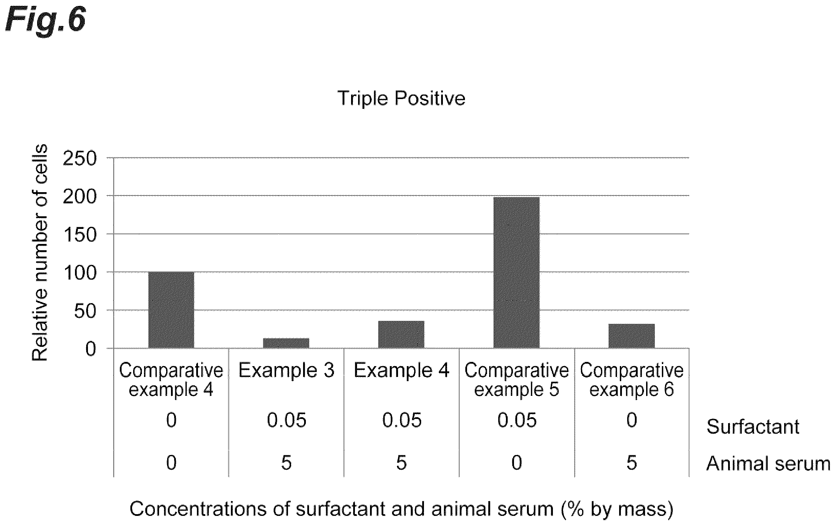

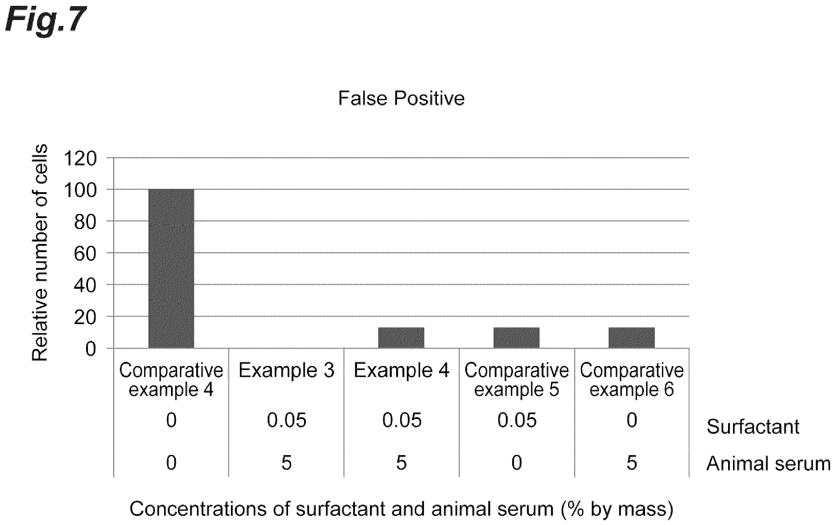

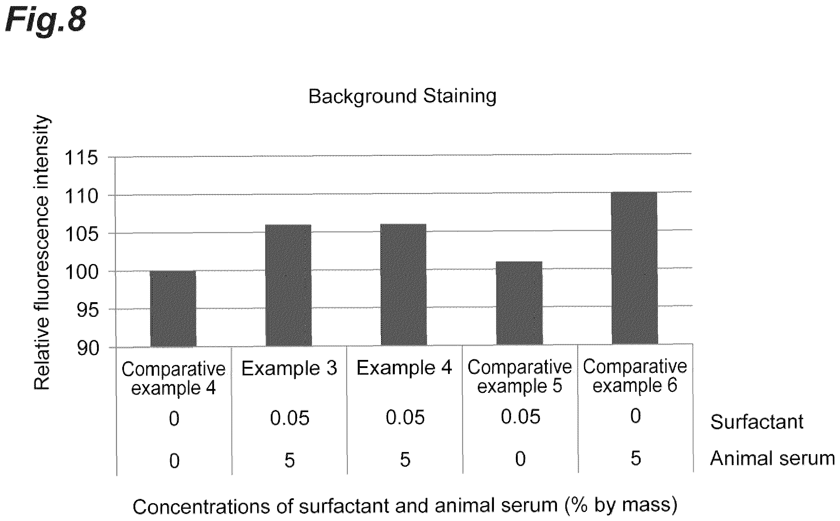

The experiments of Example 3 and Comparative Examples 4 to 6 were performed in the same way as in Example 1 and Comparative Examples 1 to 3, respectively, except that blood collected from a healthy person different from that of Test Example 1 was used. The results are shown in Table 2 and Figures 6 to 8. The relative value of the number of cells exhibiting triple positive, the relative value of the number of cells exhibiting false positive, and the relative value of the fluorescence intensity of background staining are defined as values relative to the corresponding values in Comparative Example 1, taken as 100.

### (Example 4)

Experiment was performed in the same way as in Example 3 except that the concentration of Triton X-100 in solution C was 0.1% by mass. The results are shown in Table 2 and Figures 6 to 8.

**[Table 2]**

| | Concentration (% by mass) | | Result | | | | | | |
|---|---|---|---|---|---|---|---|---|---|
| | Surfactant | Animal serum | Triple positive | | False positive | | Background staining | | Cell deformation |
| | | | Number of cells | Relative value | Number of cells | Relative value | Fluorescence intensity | Relative value | |
| Comparative Example 4 | 0 | 0 | 124 | - | 16 | - | 207.01 | - | A |
| Example 3 | 0.05 | 5 | 16 | 13 | 0 | 0 | 219.73 | 106 | A |
| Example 4 | 0.1 | 5 | 45 | 36 | 2 | 13 | 219.78 | 106 | B |
| Comparative Example 5 | 0.05 | 0 | 245 | 198 | 2 | 13 | 209.9 | 101 | A |
| Comparative Example 6 | 0 | 5 | 40 | 32 | 2 | 13 | 226.83 | 110 | A |

When the animal serum and the surfactant were reacted with the cells (examples 3 and 4), triple positives and false positives were reduced compared to Comparative Example 4. In addition, background staining was reduced, and the deformation of cells was not observed (Example 3), or even when the deformation was observed, it was so slight deformation that the cells were observed without any problem (Example 4). Meanwhile, when only either one of the animal serum and the surfactant was reacted (Comparative Examples 5 and 6), triple positives was not sufficiently reduced (Comparative Example 5), or the staining intensity of background staining increased greatly (Comparative Example 6).

### <Test Example 3>

### (Examples 5 to 7)

The same experiment as that of Example 1 was performed using blood collected from a healthy human different from that of Test Example 1. In Example 5, a solution C containing 5% by mass of the mouse serum and 0, 0.05, 0.1 or 0.2% by mass of Triton X-100 was used. In Example 6 and Example 7, experiments were performed in a similar way as in Example 5, using octyl glucoside and Tween 20 as the surfactant in solution C, respectively, instead of Triton X-100. The relative value of the number of cells exhibiting triple positive, the relative value of the number of cells exhibiting false positive and the relative value of the fluorescence intensity of background staining are defined as values relative to the result of the experiment where the concentration of the surfactant in solution C is 0% by mass, taken as 100.

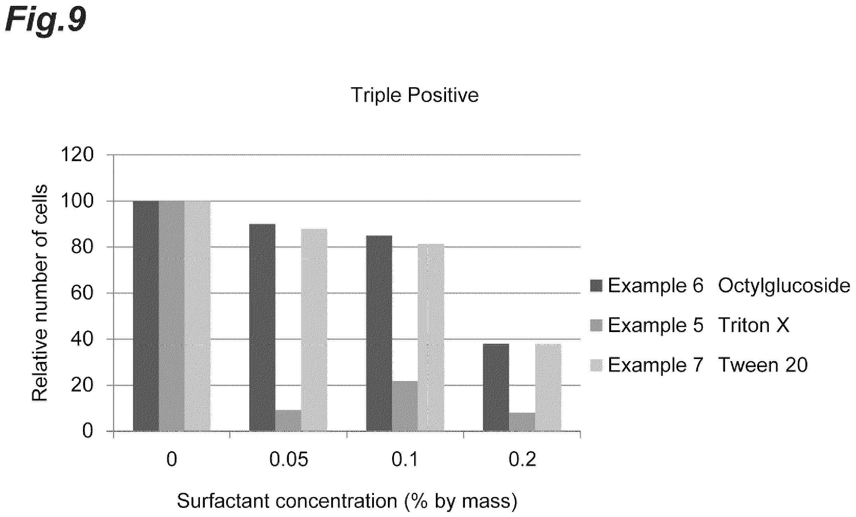

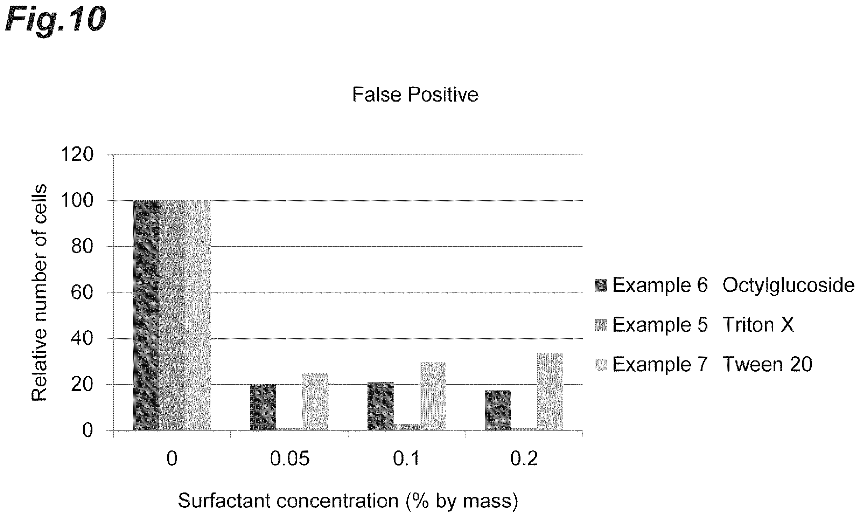

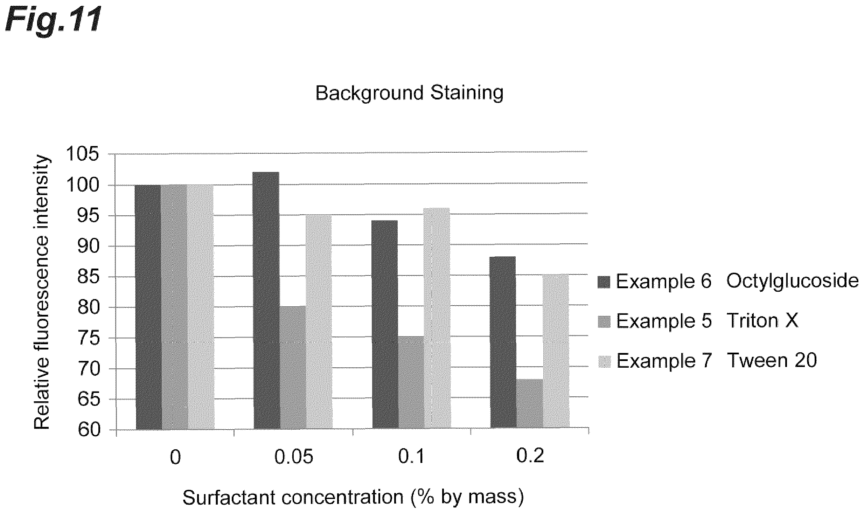

The results are shown in Figures 9 to 11. When Triton X-100 was used as a surfactant (Example 5), triple positives, false positives and background staining were reduced further than those when other surfactants were used (examples 6 and 7).

### <Test Example 4>

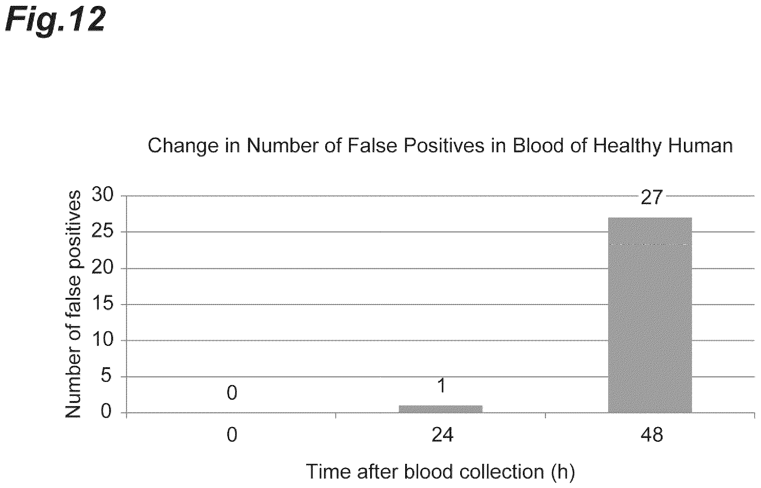

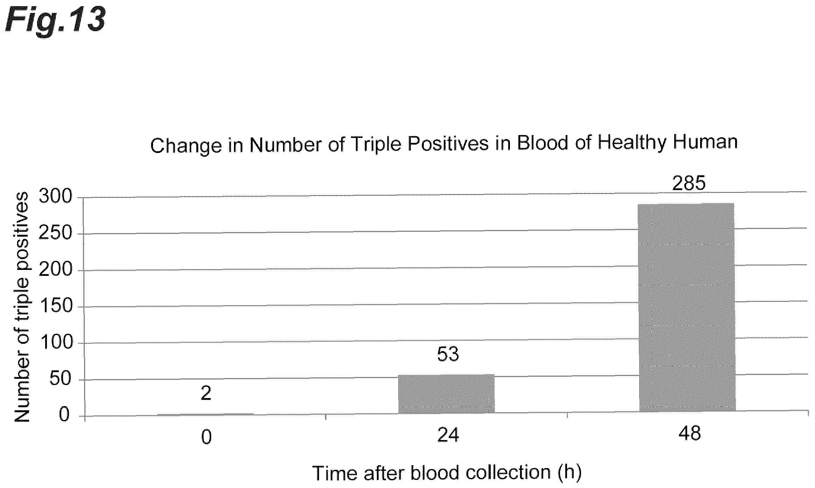

Experiment was performed in the same procedure as in Comparative Example 1 using the blood of a healthy human 0 hours, 24 hours or 48 hours after blood collection, and fluorescences emitted from fluorescent dyes were observed to determine the number of triple positives and the number of false positives. Consequently, it was shown that the number of triple positives and the number of false positives increased as the time from blood collection became longer as shown in Figure 12 and Figure 13.

### Reference Signs List

100: CTC-capturing cartridge, 105: filter, 106: through pores, 110: upper member, 115: lower member, 120: case, 125: inflow pipe, 130: inflow port, 135: outflow pipe, 140: outflow port.

## Claims

1. A method for detecting circulating tumor cells in a blood sample, comprising steps of:
(a) filtering a blood sample through a filter to capture cells on the filter;
(b) contacting with the cell-captured filter, a primary antibody that recognizes a leukocyte marker protein, and subsequently a secondary antibody that recognizes the primary antibody and is labeled with a first fluorescent dye;
(c) contacting
(c1) an animal serum,
(c2) a surfactant and
(c3) an antibody that recognizes an epithelial cell marker protein and is labeled with a second fluorescent dye
with the cell-captured filter simultaneously or in any order;
(d) contacting a third fluorescent dye that stains nucleic acids with the cell-captured filter; and
(e) irradiating the cell-captured filter with respective excitation lights of the first, second and third fluorescent dyes to detect respective fluorescences of the first, second and third fluorescent dyes emitted from each cell captured on the filter,
wherein step (d) is performed at any stage between step (a) and step (e).

2. A method for detecting circulating tumor cells in a blood sample, comprising steps of:
(a) filtering a blood sample through a filter to capture cells on the filter;
(b) contacting an antibody that recognizes a leukocyte marker protein and is labeled with a first fluorescent dye with the cell-captured filter;
(c) contacting
(c1) an animal serum,
(c2) a surfactant and
(c3) an antibody that recognizes an epithelial cell marker protein and is labeled with a second fluorescent dye
with the cell-captured filter simultaneously or in any order;
(d) contacting a third fluorescent dye that stains nucleic acids with the cell-captured filter; and
(e) irradiating the cell-captured filter with respective excitation lights of the first, second and third fluorescent dyes to detect respective fluorescences of the first, second and third fluorescent dyes emitted from each cell captured on the filter,
wherein step (d) is performed at any stage between step (a) and step (e).

3. The method according to claim 1 or 2, wherein step (c) and step (d) are performed simultaneously to contact (c1), (c2), (c3) and the third fluorescent dye that stains nucleic acids simultaneously with the cell-captured filter.

4. The method according to any one of claims 1 to 3, wherein the surfactant comprises a nonionic surfactant.

5. The method according to any one of claims 1 to 3, wherein the surfactant comprises polyoxyethylene octyl phenyl ether.

6. The method according to any one of claims 1 to 5, wherein a concentration of the surfactant is 0.05% by mass to 0.2% by mass.

7. The method according to any one of claims 1 to 6, wherein an animal from which the animal serum derives, an animal from which the primary antibody or the antibody that recognizes a leukocyte marker protein derives, and an animal from which the antibody that recognizes an epithelial cell marker protein derives are the same animal.

8. The method according to claim 7, wherein the animal is a mouse.

9. The method according to any one of claims 1 to 8, wherein a concentration of the animal serum is 2% by mass to 10% by mass.

10. The method according to any one of claims 1 to 9, wherein the leukocyte marker protein is CD45.

11. The method according to any one of claims 1 to 10, wherein the epithelial cell marker protein is cytokeratin or a tumor marker.

12. A pretreatment method for detecting circulating tumor cells on a cell-captured filter, comprising a step of contacting an animal serum and a surfactant with the cell-captured filter simultaneously or in any order.

13. The method according to claim 12, wherein the cell-captured filter is treated with a primary antibody that recognizes a leukocyte marker protein and a secondary antibody that recognizes the primary antibody and is labeled with a first fluorescent dye, prior to the step of contacting the animal serum and the surfactant.

14. The method according to claim 12, wherein the cell-captured filter is treated with an antibody that recognizes a leukocyte marker protein and is labeled with a first fluorescent dye, prior to the step of contacting the animal serum and the surfactant.

15. The method according to claim 13 or 14, wherein the animal serum, the surfactant, an antibody that recognizes an epithelial cell marker protein and is labeled with a second fluorescent dye, and a third fluorescent dye that stains nucleic acids are simultaneously contacted with the cell-captured filter.

16. The method according to any one of claims 12 to 15, wherein the surfactant comprises a nonionic surfactant.

17. The method according to any one of claims 12 to 15, wherein the surfactant comprises polyoxyethylene octyl phenyl ether.

18. The method according to any one of claims 12 to 17, wherein a concentration of the surfactant is 0.05% by mass to 0.2% by mass.

19. The method according to claim 15, wherein an animal from which the animal serum derives, an animal from which the primary antibody or the antibody that recognizes a leukocyte marker protein derives, and an animal from which the antibody that recognizes an epithelial cell marker protein derives are the same animal.

20. The method according to claim 19, wherein the animal is a mouse.

21. The method according to any one of claims 12 to 20, wherein a concentration of the animal serum is 2% by mass to 10% by mass.

22. The method according to claim 13 or 14, wherein the leukocyte marker protein is CD45.

23. The method according to claim 15, wherein the epithelial cell marker protein is cytokeratin or a tumor marker.
